# EUROPÄISCHE PATENTSCHRIFT

(11) **EP 2 265 230 B1**
(45) Veröffentlichungstag und Bekanntmachung des Hinweises auf die Patenterteilung: **09.05.2012**
(21) Anmeldenummer: 09731101.3
(22) Anmeldetag: 24.03.2009
(51) Int. Cl.: A61F 2/14, A61F 2/16, A61L 27/50, A61F 9/00

(54) **OPHTHALMOLOGISCHES IMPLANTAT MIT MARKIERUNG ZU DESSEN DETEKTION UND/ODER IDENTIFIKATION**
OPHTHALMOLOGIC IMPLANT HAVING A MARKER FOR DETECTION AND/OR IDENTIFICATION
IMPLANT OPHTALMOLOGIQUE COMPRENANT UN MARQUEUR DE DÉTECTION ET/OU D'IDENTIFICATION

(30) Priorität: 07.04.2008 DE 102008017592
(43) Veröffentlichungstag der Anmeldung: 29.12.2010
(73) Patentinhaber: Carl Zeiss Meditec AG, 07745 Jena (DE)
(72) Erfinder: WIECHMANN, Martin, 07743 Jena (DE); GERLACH, Mario, 16540 Hohen Neuendorf (DE)
(74) Vertreter: Lechner, Armin Anton
(86) Internationale Anmeldenummer: PCT/EP2009/053453
(87) Internationale Veröffentlichungsnummer: WO 2009/124838

(56) Entgegenhaltungen:
- EP-A- 0 402 825
- EP-A2- 1 225 441
- WO-A2-2007/120755
- GB-A- 2 100 878
- US-A- 4 490 860
- US-A- 4 632 773
- US-A- 4 743 255
- US-A1- 2004 032 566
- US-A1- 2006 050 146
- US-A1- 2006 235 428
- US-A1- 2007 171 363
- VON DEFFER H; MERTZ M; WARTH P; KÖHLER H W: "Infrared diagnosis in ophthalmology" FORTSCHRITTE DER MEDIZIN, Bd. 96, Nr. 20, 25. Mai 1978 (1978-05-25), Seiten 1053-1057, XP009121167 germany, west

## Beschreibung

### Technisches Gebiet

Die Erfindung betrifft ein ophthalmologisches Implantat, insbesondere eine Intraokularlinse, mit mindestens einer Markierung hergestellt mit mindestens einem Farbstoff.

### Stand der Technik

Bei der Implantation von ophthalmologischen Implantaten, insbesondere bei dem Einsetzen von Intraokularlinsen in das menschliche Auge sind spezielle Beleuchtungsbedingungen notwendig um das Implantat erkennbar darstellen zu können. Dies liegt darin begründet, dass Intraokularlinsen üblicherweise hochtransparente optische Elemente darstellen und Intraokularlinsen und diejenigen Bereiche des Auges, in die die Intraokularlinse eingesetzt werden soll üblicherweise nahezu den gleichen Refraktionsindex aufweisen. Um die Lage und Position derartiger ophthalmologischer Implantate einigermaßen bestimmen zu können wird durch geeignete Beleuchtungsverfahren ein genügend großer Kontrast zwischen dem Implantat und den das Implantat umgebenden Bereichen des Auges im Operationsfeld erzielt. Hierfür wird zum Beispiel bei Katarakt-Operationen der so genannte "Red Reflex" verwendet. Dabei können zum Beispiel Linsenreste eines Patientenauges kontrastreich mit einem Mikroskop dargestellt und gesehen werden. Nachteilig an derartigen Beleuchtungen ist jedoch, dass diese nur relativ aufwändig zu realisieren sind. Eine weitere Möglichkeit zur Darstellung der Lage und Position einer Intraokularlinse innerhalb eines Patientenauges ist in der US 2004/0068317 A1 beschrieben. Dabei wird eine Intraokularlinse offenbart, deren randliche Befestigungsmittel, die so genannten Haptiken, mit permanent sichtbaren Markierungen eingefärbt sind. Aus der EP 0 402 825 A1 ist eine weitere Intraokularlinse mit Markierungen bekannt, wobei die Markierungen aus fluoreszierenden Farbstoffen hergestellt sind und die Farbstoffe innerhalb des für den Menschen sichtbaren Lichtspektrums fluoreszieren. Nachteilig an den bekannten Intraokularlinsen mit Markierungen ist jedoch, dass einerseits bei dauerhaft sichtbaren Markierungen die wirksame Pupillenöffnung des Patienten und insbesondere die Lichttransmission des Implantats im visuellen Spektralbereich deutlich eingeschränkt sind. Zusätzlich können Transmissionsmodulationen in der Nähe der Augenpupille zu unerwünschten photischen Phänomenen durch Beugungseffekte (Phasenstörung z.B. an Kanten) führen. Andererseits ergibt sich auch bei der Verwendung von Farbstoffen, die im für den Menschen sichtbaren Lichtspektrum fluoreszieren der Nachteil, dass zum Beispiel bei Sonneneinstrahlung diese Farbstoffe angeregt werden und entsprechend fluoreszieren. Dadurch ergibt sich eine deutliche Beeinträchtigung des visuellen Sichtempfindens des Implantat- bzw. Linsenträgers, da durch das Fluoreszieren eine starke Beeinflussung der Lichttransmission des Implantats bzw. der Intraokularlinse im visuellen Spektralbereich erfolgt. Zudem erfolgt eine Überlagerung des Umgebungslichtes durch das zusätzliche diffuse Licht (Fluoreszenzlicht), wodurch das visuelle Sichtempfinden aufgrund eines deutlich reduzierten Kontrastes verschlechtert wird.

Aus US 2006/235428 A1 ist ein okulares Implantat bekannt, welches Markierungen aufweist. Lediglich ganz allgemein ist dort auch offenbart, dass diese Markierung ein Farbstoff sein kann, welcher ultraviolettes Licht absorbiert und im sichtbaren Spektralbereich fluoresziert und somit im sichtbaren Spektralbereich Licht emittiert.

In VON DEFFER H; MERTZ M; WARTH P; KÖHLER H W: "Infrared diagnosis in ophthalmology" FORTSCHRITTE DER MEDIZIN, Bd. 96, Nr. 20, 25. Mai 1978 (1978-05-25), Seiten 1053-1057, XP009121167 Germany, West, ist lediglich allgemein über die Detektion von Pigmentverteilungen in der Iris des menschlichen Auges gesprochen.

### Darstellung der Erfindung

Es ist Aufgabe der vorliegenden Erfindung ein ophthalmologisches Implantat der eingangs genannten Art bereitzustellen, dessen Lage und Position sicher detektiert werden kann und/oder dessen Identifikation ohne eine Beeinträchtigung des visuellen Sichtempfindens eines Implantatträgers möglich ist.

Diese Aufgabe wird durch ein ophthalmologisches Implantat gemäß dem Anspruch 1 gelöst. Vorteilhafte Ausgestaltungen der Erfindung sind in den jeweiligen Unteransprüchen angegeben.

Ein erfindungsgemäßes ophthalmologisches Implantat, insbesondere eine Intraokularlinse, umfasst mindestens eine Markierung, die mit mindestens einem Farbstoff hergestellt ist, wobei der Farbstoff ein fluoreszierender Farbstoff mit einem Emissionsmaximum außerhalb des für den Menschen sichtbaren Lichtspektrums oder ein absorbierender Farbstoff mit einem Absorptionsmaximum außerhalb des für den Menschen sichtbaren Lichtspektrums ist, jeweils ohne die Lichttransmission des Implantats im visuellen Spektralbereich, der zwischen ca. 380 nm und 780 nm oder einem Teilbereich des visuellen Spektralbereichs zwischen ca. 450 nm und 750 nm, liegt, wesentlich zu beeinflussen. Dadurch ist es möglich, permanente Markierungen an dem ophthalmologischen Implantat anzuordnen die einerseits die Lichttransmission des Implantats im visuellen Spektralbereich nicht beeinflussen und andererseits außerhalb des für den Menschen sichtbaren Lichtspektrums fluoreszieren oder absorbieren, so dass bei einer entsprechenden Beleuchtung des Implantats eine zuverlässige und sichere Detektion der Lage und Position und/oder eine Identifikation des Implantats anhand der Markierung ohne eine Beeinflussung oder Beeinträchtigung des visuellen Sichtempfindens des Implantatträgers ermöglicht wird. Zudem ist eine zweifelsfreie Bestimmung einer Vorder- und Rückseite des Implantats mittels geeigneter Markierungen, wie zum Beispiel asymmetrische Formen und Figuren, Buchstaben, Zahlen oder Texte möglich. Des Weiteren ist es vorteilhafterweise möglich, das Implantat bzw. die enthaltene Markierung bei einem geeigneten Farbstoff mit weißem Licht oder mit einem Licht eines geeigneten Spekt ralbereichs zu bestrahlen und hierdurch eine entsprechende Fluoreszenz oder eine Absorption des Farbstoffs bzw. der Markierung außerhalb des für den Menschen sichtbaren Lichtspektrums zu bewirken. Aufwändige Beleuchtungseinrichtungen sind nicht notwendig. Es ist aber auch möglich, dass ein Anregungslicht für den Farbstoff der Markierung mit einem vorbestimmten Wellenlängenbereich verwendet wird.

Der fluoreszierende Farbstoff ist aus einer der Strukturklassen die Cyanine, Mercocyanine, Phtalocyanine, Rhodamine, Porphyrine, Chlorophylle, Triarylmethane, Xanthene, Oxazine, Thiazine, Safranine, heterozyklische Pyrilium-und Thiapyriliumsalze, Perrylene, Terrylene, Quaterrylene, Quaternele, Azofarbstoffe, Metallkomplexe oder Chinone umfasst, ausgewählt. Es ist aber auch möglich, dass der lichtabsorbierende Farbstoff ein UV-Absorber aus einer der Strukturklassen die Benzophenone, Benzotriazole, Salicylsäureester, Resorcinmnobenzonate, Oxalsäureanilide, Zimtsäureesterderivate, p-Hydroxybenzoesäureester umfasst und/oder ein IR-Absorber aus einer dieser Strukturklassen, ist. Auch andere fluoreszierende oder absorbierende Farbstoffe sind zur Herstellung der Markierungen verwendbar. Entscheidend dabei ist jedoch, dass der Farbstoff außerhalb des für den Menschen sichtbaren Lichtspektrums fluoresziert oder absorbiert. Des Weiteren darf der Farbstoff nicht zu einer sichtbaren Eintrübung oder einer das Sichtempfinden des Implantatträgers signifikant beeinflussenden oder zu einer deutlich schlechteren Lichttransmission führenden Verfärbung des ophthalmologischen Implantats führen. Vorteilhafterweise ist der Farbstoff zudem bioverträglich und stabil gegenüber den Umgebungsbedingungen im menschlichen Auge, definiert durch das Umgebungsmedium, die Temperatur und die Lichteinwirkung im UV/VIS/IR-Bereich. Zudem muss der Farbstoff gegenüber einer Ausspülung durch zum Beispiel das Kammerwasser des Auges resistent sein. Die Farbstoffmarkierung kann auch gegenüber dem das Implantat umgebenden Bereich zum Beispiel mittels einer transparenten Folie oder einer Beschichtung abgedichtet bzw. abgeschirmt sein. Bei der Verwendung von hydrophoben und hydrophilen Acrylatmaterialien zur Herstellung des ophthalmologischen Implantats muss der Farbstoff in diese Materialien einbindbar oder anheftbar sein. Die im Vorhergehenden beschriebenen Farbstoffkriterien müssen dabei insbesondere durch die Farbstoffe bzw. Markierungen in dem an das Implantat gebundenen Zustand erfüllt sein. Geeignete Farbstoffe, die im infraroten Spektralbereich fluoreszieren weisen zum Beispiel eine Fluoreszenzwellenlänge zwischen 820 nm und 1100 nm auf. Die Anregungswellenlängen liegen dabei zwischen 750 nm und 850 nm. Beispielhaft für einen derartigen fluoreszierenden Farbstoff ist Indocyaningrün zu nennen, dessen Anregungswellenlänge zwischen 700 nm und 800 nm und dessen Fluoreszenzwellenlängen zwischen 805 nm und 950 nm bei einem Emissionsmaximum bei 835 nm liegen. Weitere geeignete und im Infrarotbereich fluoreszierende Farbstoffe sind unter den Substanznamen NIR1, NIR820, SNIR2 und SNIR4 bekannt.

Da ophthalmologische Implantate in vielen Anwendungsbereichen aus hydrophoben oder hydrophilen Acrylatmaterialien bestehen sind die für das erfindungsgemäße Implantat verwendbaren Farbstoffe in diese Acrylatmaterialien einbindbar oder anheftbar. Insbesondere hat sich das Material Polymethyl-Methacrylat (PMMA) als geeignet für ophthalmologische Implantate, insbesondere Intraokularlinsen erwiesen. Das Implantat kann aber auch aus soften hydrophilen Co-Polymeren bestehend aus mindestens einem hydrophilen Monomer (z.B. HEMA) und mindestens einem hydrophoben Monomer z.B. einem Alkoxyalkyl-Methacrylat-Monomer wie z.B. EOEMA oder MMA. Auch Kombinationen mit anderen (Meth-)Acrylat-Monomeren sowie Kombinationen aus HEMA + Collagenverbindungen sind denkbar.

Das Implantat kann aber auch aus soften hydrophoben Co-Polymeren bestehend aus mindestens zwei hydrophoben Monomerkomponenten mit unterschiedlicher Glasübergangstemperatur z.B. Phenylacrylat + Phenylmethacrylat bestehen.

Zudem kann als Implantatmaterial Silikon verwendet werden.

Durch die erfindungsgemäßen ophthalmologischen Implantate ist es möglich, die Lage und Position zum Beispiel einer Intraokularlinse während oder nach einem chirurgischen Eingriff mittels eines geeigneten Mikroskopiesystems sicher und zuverlässig darzustellen und zu bestimmen. Des Weiteren ergibt sich eine zuverlässige Identifikationsmethode zur Identifizierung des Implantats, da neben Lage-und Positionsdaten auch herstellungsspezifische Daten des Implantats, wie zum Beispiel optische Daten oder das Herstellungsdatum angezeigt und ermittelt werden können.

In weiteren vorteilhaften Ausgestaltungen des erfindungsgemäßen Implantats ist die mindestens eine Markierung an mindestens einem Befestigungselement und/oder einem Linsenkörper und/oder einem Rand des Linsenkörpers des ophthalmologischen Implantats ausgebildet. Die Markierung kann dabei als geometrische Form, insbesondere als Punkt, Strich, Kreuz, Kreis, Halbkreis, Oval, Quadrat, Rechteck, Vieleck oder einer Kombination der geometrischen Formen ausgebildet sein. Auch andere geometrische Formen sind denkbar. Des Weiteren ist es möglich, dass die Markierung aus Buchstaben und/oder Ziffern und/oder einem Barcode zur Speicherung und Darstellung von implantatspezifischen Daten besteht. Des Weiteren ist es möglich, dass die mindestens eine Markierung mindestens ein Befestigungselement und/oder einen Linsenkörper des ophthalmologischen Implantats voll- oder teilflächig markiert. Eine weitere vorteilhafte Ausgestaltung des erfindungsgemäßen Implantats besteht darin, dass dieses mindestens zwei mittels unterschiedlicher Farbstoffe hergestellte Markierungen aufweist. Durch die Verwendung unterschiedlicher Farbstoffe ist eine besonders einfache und schnelle Lage- und Positionsbestimmung des Implantats im Auge möglich. Auch die Anordnung von mehreren Markierungen in einem oder mehreren geometrischen Mustern erleichtert die Ermittlung der Lage und Position des Implantats innerhalb des Auges.

In weiteren vorteilhaften Ausgestaltungen des erfindungsgemäßen Implantats wird die Markierung während der Herstellung und/oder nach der Fertigstellung des ophthalmologischen Implantats ausgebildet. Dabei ist es möglich, dass der die Markierung ausbildende Farbstoff vor und/oder nach einer Polymerisation oder Vulkanisation eines Linsenmaterials des ophthalmologischen Implantats in dieses eingebracht ist und/oder nach der Polymerisation oder Vulkanisation des Linsenmaterials auf eine Oberfläche des ophthalmologischen Implantats aufgebracht wird. Es ist aber auch möglich, dass auch nach einer Polymerisation oder Vulkanisation des Linsenmaterials des Implantats der Farbstoff zur Ausbildung der Markierung in das Innere des Implantatkörpers injiziert wird. Werden die Markierungen an der Oberfläche des ophthalmologischen Implantats aufgebracht, so ist es möglich, dass zumindest diejenigen Bereiche der Oberfläche des Implantats an denen die Markierung oder die Markierungen angeordnet sind mit einer Schutzfolie oder einer schützenden Beschichtung überzogen sind. Eine derartige Ausbildung einer Schutzfolie verhindert zuverlässig eine mögliche Ausspülung des Farbstoffs bzw. der Markierung durch das Kammerwasser des Auges. Besteht das erfindungsgemäße ophthalmologische Implantat aus einem hydrophoben oder hydrophilen Acrylatmaterial so ist es zum Beispiel möglich, den zentralen und üblicherweise eine Optik aufweisenden Bereich des Implantats, insbesondere der Intraokularlinse mit einem durchsichtigen, d. h. einem Acrylatmaterial mit durchsichtigen, d. h. einem Acrylatmaterial mit einer hohen Lichttransmission auszubilden. Um diesen Bereich kann dann Acrylatmaterial gegossen werden, welches den die Markierung ausbildenden Farbstoff enthält. Es ist aber auch möglich, dass nur die Befestigungselemente, nämlich die Haptiken der Implantate die Markierung oder die Markierungen aufweisen. Die Befestigungsmittel können dabei wiederum aus einem den Farbstoff und damit die Markierung aufweisenden Material bestehen und an dem Linsenkörper nachträglich befestigt werden. Des Weiteren ist es möglich, die Markierungen zum Beispiel durch ein Präge- oder Druckverfahren auf dem Implantat anzuordnen. Weitere Verfahren, die bei erfindungsgemäßen Implantaten aus Kunststoff angewandt werden können sind beispielsweise Mehrfarben-/Mehrkomponentenspritzguss-Verfahren, Hinterspritz- und Zwischenschichtspritz-Verfahren, so genannte In-Mould-Dekorierverfahren bzw. Labeling-Verfahren, eine Co- oder Mehrschichten-Extrusion, Laminieren und Plasmaverfahren wie zum Beispiel Chemical Vapor Deposition (CVD).

In weiteren vorteilhaften Ausgestaltungen des erfindungsgemäßen Implantats ist die mindestens eine Markierung auf einer Folie aufgebracht, wobei die Folie in das ophthalmologische Implantat eingebracht oder auf eine Oberfläche des ophthalmologischen Implantats aufgebracht werden kann. Durch die Verwendung einer Folie können die Markierungen relativ einfach im Druckverfahren hergestellt werden. Wird die Folie auf das Implantat aufgebracht, so erfolgt dies üblicherweise mittels einer Klebeverbindung. Ein Mikroskopiesystem zur Detektion der Lage und Position und/oder zur Identifikation eines wie im Vorhergehenden beschriebenen ophthalmologischen Implantats, insbesondere einer Intraokularlinse umfasst ein Mikroskop mit einer Mikroskopoptik, mindestens ein Beleuchtungssystem zur Beleuchtung und Anregung von mindestens einer Markierung des in einem Auge angeordneten ophthalmologischen Implantats, mindestens eine Kamera zur Aufnahme der außerhalb des für den Menschen sichtbaren Lichtspektrums emittierenden fluoreszierenden oder absorbierenden Markierung und mindestens eine Auswerteeinheit für mindestens ein von der Kamera aufgenommenes Bild der Markierung. Das Mikroskopiesystem ermöglicht eine zuverlässige und sichere Detektion der Lage und Position und/oder Identifikation des ophthalmologischen Implantats. Dabei umfasst das Beleuchtungssystem mindestens eine Lichtquelle, wobei die Lichtquelle weißes Licht oder ein Anregungslicht für den Farbstoff der Markierung in einem vorbestimmten Wellenlängenbereich abstrahlt. Die Art der Beleuchtung richtet sich dabei nach der Art des verwendeten Farbstoffes, die für die Markierung geeigneten Farbstoffe wurden im Vorhergehenden bereits ausführlich beschrieben. Die von der Kamera aufgenommenen Bilddaten werden in der Auswerteeinheit für eine Lage- und Positionsbestimmung und/oder eine Identifikation des Implantats ausgewertet. Dabei kann die Auswerteeinheit mindestens ein Koppelungssystem zum Einkoppeln der aufgenommenen Bilder der Markierung in die Mikroskopoptik des Mikroskops aufweisen. Dadurch ist es möglich, dass die von der Kamera aufgenommenen Bilddaten für eine Betrachtung durch einen Benutzer des Mikroskopiesystems mit den Bildern des Objektbereichs, d. h. des ophthalmo logischen Implantats und der es umgebenden Strukturen in Überlagerung gebracht wird. Auch dies dient zu einer exakten Lage- und Positionskontrolle des Implantats. Des Weiteren ist es möglich, dass die Auswerteeinheit mindestens ein Identifikationssystem zur Identifikation von durch die Markierung übermittelnden Informations- und I-dentifikationsdaten des ophthalmologischen Implantats umfasst. Die so ermittelten Daten können dabei wiederum über das Koppelungssystem in die Mikroskopoptik des Mikroskops optisch eingeblendet werden, so dass sie durch den Benutzer betrachtet werden können. Des Weiteren ist es möglich, die ermittelten Informations- und Identifikationsdaten zum Beispiel an eine separate Anzeigevorrichtung wie einem Bildschirm oder auch einen Drucker weiterzuleiten. In einer weiteren vorteilhaften Ausgestaltung des Mikroskopiesystems umfasst die Auswerteeinheit mindestens ein automatisches Nachführsytem zur Erkennung von Bewegungen und/oder Lage- und Positionsänderungen der Markierung und des ophthalmologischen Implantats. Die Auswerteeinheit kann zudem mindestens ein Autofokussystem umfassen, wobei die Fokussierung der Mikroskopoptik auf das ophthalmologische Implantat mittels der von der Kamera aufgenommenen Bilder der Markierung und deren Auswertung erfolgt.

In einer weiteren vorteilhaften Ausgestaltung des Mikroskopiesystems ist das Beleuchtungssystem als Spaltbeleuchtungssystem ausgebildet. Ein derartiges Spaltbeleuchtungssystem ermöglicht es, dass die Position des ophthalmologischen Implantats nach dem chirurgischen Eingriff besser kontrolliert werden kann. So muss die Pupille bei der Kontrolle nicht künstlich zum Bei spiel unter Verwendung von geeigneten Augentropfen erweitert werden, was wiederum dazu führt, dass die Befestigungselemente des Implantats in keinem Fall sichtbar sind.

Ein optisches Nachweisverfahren zur Detektion der Lage und Position und/oder der Identifikation eines wie im Vorhergehenden beschriebenen ophthalmologischen Implantats, insbesondere einer Intraokularlinse umfasst folgende Schritte: a) Beleuchten des ophthalmologischen Implantats mit mindestens einer Lichtquelle eines Beleuchtungssystems, wobei die Lichtquelle weißes Licht oder ein Anregungslicht für einen Farbstoff der Markierung des Implantats in einem vorbestimmten Wellenlängenbereich abstrahlt; b) Aufnahme der außerhalb des für den Menschen sichtbaren Lichtspektrums emittierenden fluoreszierenden oder absorbierenden Markierung mittels einer Kamera; c) Auswertung von mindestens einem von der Kamera aufgenommenen Bild der Markierung mittels mindestens einer Auswerteeinheit. Durch das Nachweisverfahren ist eine zuverlässige und sichere Detektion der Lage und Position und/oder Identifikation des ophthalmologischen Implantats gewährleistet. Zudem ist gewährleistet, dass die Anwendung des Verfahrens keine Beeinträchtigung des visuellen Sichtempfindens eines Implantatträgers bewirkt, da die durch weißes Licht oder ein Anregungslicht in einem vorbestimmten Wellenbereich bestrahlte Markierung außerhalb des für den Menschen sichtbaren Lichtspektrums emittiert oder absorbiert. In weiteren vorteilhaften Ausgestaltungen des Nachweisverfahrens umfasst die Auswertung gemäß Verfahrensschritts c) ein Einkoppeln des aufgenommenen Bilds der Markierung in eine Mikroskopoptik eines Mikroskops. Dadurch ist es möglich, ein Überlagerungsbild bestehend aus dem von der Kamera aufgenommenen Bild der Markierung und der Abbildung eines Objektbereichs, nämlich des beobachteten Implantats und dessen Umgebung für einen Betrachter bereitzustellen. Es ist aber auch möglich, dass die Auswertung gemäß Verfahrensschritt c) eine Identifikation von durch die Markierung übermittelte I-dentifikationsdaten des ophthalmologischen Implantats und/oder die Darstellung von Informationsdaten bezüglich des Implantats wie zum Beispiel dessen korrekter Positionierung innerhalb des Auges umfasst. Die Identifikationsdaten können dabei optische Daten oder auch Daten bezüglich des Materials des Implantats oder dessen Herstellung beinhalten. Des Weiteren ist es möglich, dass die Auswertung gemäß Verfahrensschritt c) eine Erkennung von Bewegungen und/oder Lage- und Positionsänderungen der Markierung und des ophthalmologischen Implantats umfasst. Des Weiteren kann die Auswertung gemäß Verfahrensschritt c) eine Fokussierung einer Mikroskopoptik auf das ophthalmologische Implantat mittels der von der Kamera aufgenommenen Bilder der Markierung umfassen. Dadurch wird zum Beispiel ein optimales Bildergebnis bei der Aufnahme der Markierungen durch die Kamera erzielt.

### Kurze Beschreibung der Zeichnungen

Ausführungsbeispiele der Erfindung werden nachfolgend anhand schematischer Zeichnungen näher erläutert. Es zeigen:
- Fig. 1: eine schematische Darstellung eines erfindungsgemäßen ophthalmologischen Implantats gemäß einer ersten Ausführungsform;
- Fig. 2: eine schematische Darstellung eines erfindungsgemäßen ophthalmologischen Implantats gemäß einer zweiten Ausführungsform;
- Fig. 3: eine schematische Darstellung eines erfindungsgemäßen ophthalmologischen Implantats gemäß einer dritten Ausführungsform;
- Fig. 4: eine schematische Darstellung eines erfindungsgemäßen ophthalmologischen Implantats gemäß einer vierten Ausführungsform;
- Fig. 5: eine schematische Darstellung eines erfindungsgemäßen ophthalmologischen Implantats gemäß einer fünften Ausführungsform;
- Fig. 6: eine schematische Darstellung eines erfindungsgemäßen ophthalmologischen Implantats gemäß einer sechsten Ausführungsform;
- Fig. 7: eine schematische Darstellung eines erfindungsgemäßen ophthalmologischen Implantats gemäß einer siebten Ausführungsform;
- Fig. 8: eine schematische Darstellung eines erfindungsgemäßen ophthalmologischen Implantats gemäß einer achten Ausführungsform;
- Fig. 9: eine schematische Darstellung eines erfindungsgemäßen ophthalmologischen Implantats gemäß einer neunten Ausführungsform; und

- Fig. 10: eine schematische Darstellung eines Mikroskopiesystems.

### Bevorzugte Ausführung der Erfindung

In den Figuren werden gleiche oder funktionsgleiche Elemente mit den gleichen Bezugszeichen versehen.

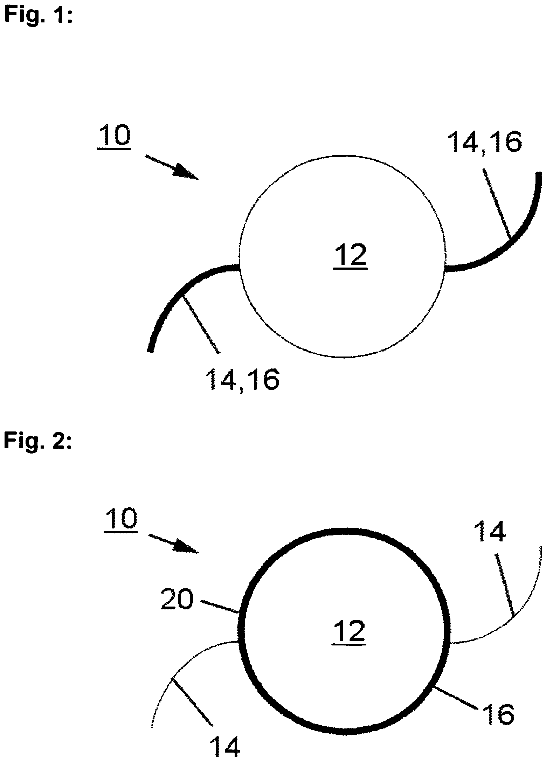

In einer schematischen Darstellung ist in Fig. 1 eine erste Ausführungsform eines ophthalmologischen Implantats, nämlich einer Intraokularlinse 10 gemäß einer ersten Ausführungsform dargestellt. Die Intraokularlinse 10 besteht aus einem Linsenkörper 12, der als optische Einrichtung ausgebildet ist oder eine entsprechende optische Einrichtung aufweist (nicht dargestellt). An einem Rand 20 des Linsenkörpers 12 sind Befestigungselemente 14, so genannte Haptiken ausgebildet. Üblicherweise besteht der Linsenkörper 12 wie auch die Befestigungselemente 14 aus einem hydrophoben oder hydrophilen Acrylatmaterial. Insbesondere bestehen der Linsenkörper 12 und die Befestigungselemente 14 aus Polymethyl-Methacrylat (PMMA) oder soften Co-Polymeren bestehend aus einem hydrophilen Monomer und einem Alkoxyalkyl-Methacrylat-Monomer. Der Linsenkörper 12 ist dabei derart ausgebildet, dass das verwendete Material die Lichttransmission des Implantats bzw. der Intraokularlinse 10 im visuellen Spektralbereich zwischen ca. 380 nm und ca. 780 nm nicht wesentlich beeinflusst. Eine derartige Ausgestaltung des Implantats bzw. der Intraokularlinse 10 wird auch bei den in den Figuren 2 bis 9 dargestellten Ausführungsformen verwendet. Es ist aber auch möglich, dass das verwendete Material die Lichttransmission des Implantats bzw. der Intraokularlinse 10 nur in einem Teilbereich des visuellen Spektralbereichs zwischen ca. 450 nm und ca. 750 nm nicht wesentlich beeinflusst. So kann das Implantat bzw. die Intraokularlinse 10 ein leichte Gelbfärbung aufweisen, die als UV-Schutz dient, wobei die Absorptionskanten des UV-Schutzes zwischen ca. 400 nm und 450 nm liegen.

Des Weiteren erkennt man, dass in dem dargestellten ersten Ausführungsbeispiel Markierungen 16 an dem jeweiligen Befestigungselement 14 ausgebildet sind. Dabei sind die Markierungen 16 jeweils vollflächig an dem entsprechenden Befestigungselement 14 angeordnet. Die Markierungen 16 sind dabei jeweils aus einem fluoreszierenden Farbstoff mit einem Emissionsmaximum außerhalb des für den Menschen sichtbaren Lichtspektrums von üblicherweise 380 nm bis 780 nm hergestellt. Es ist aber auch möglich, dass zur Herstellung der Markierung 16 ein absorbierender Farbstoff mit einem Absorptionsmaximum außerhalb des für den Menschen sichtbaren Lichtspektrums verwendet wird. In dem in Fig. 1 wie auch den Fig. 2 bis 9 dargestellten Ausführungsbeispielen der Intraokularlinse 10 wird der fluoreszierende Farbstoff Indocyaningrün verwendet, dessen Anregungswellenlänge zwischen 700 nm und 800 nm und dessen Fluoreszenzwellenlängen zwischen 805 nm und 950 nm bei einem Emissionsmaximum bei 835 nm liegen. Indocyaningrün wird bisher zur mikroskopischen Untersuchung von mit diesem Farbstoff angereicherten Gewebe verwendet. Dabei können Gewebebereiche identifiziert werden, in denen dieser Fluoreszenzfarbstoff angereichert ist. Vorteilhafterweise weist Indocyaningrün eine nur sehr geringe Toxizität auf, da es im menschlichen Körper über die Leber und Gallenwege ausgeschieden und nicht von der Darm-Schleimhaut resorbiert wird.

Neben der in der Fig. 1 dargestellten vollflächigen Ausbildung der Markierung 16 an dem Befestigungselement 14 ist es auch möglich, die Markierung 16 teilflächig an dem Befestigungselement 14 auszubilden (nicht dargestellt). Dadurch kann die Lage des Befestigungselementes 14 während der Positionierung des Implantats 10 im Auge deutlich erkannt werden.

Fig. 2 zeigt eine schematische Darstellung eines ophthalmologischen Implantats 10, nämlich einer Intraokularlinse 10 gemäß einer zweiten Ausführungsform. Man erkennt, dass ein Randbereich 20 des Linsenkörpers 12 mit einer ringförmigen Markierung 16 versehen ist. Die Intraokularlinse 10 weist wiederum zwei Befestigungselemente 14 auf, die jedoch in diesem Ausführungsbeispiel ohne Markierungen versehen sind.

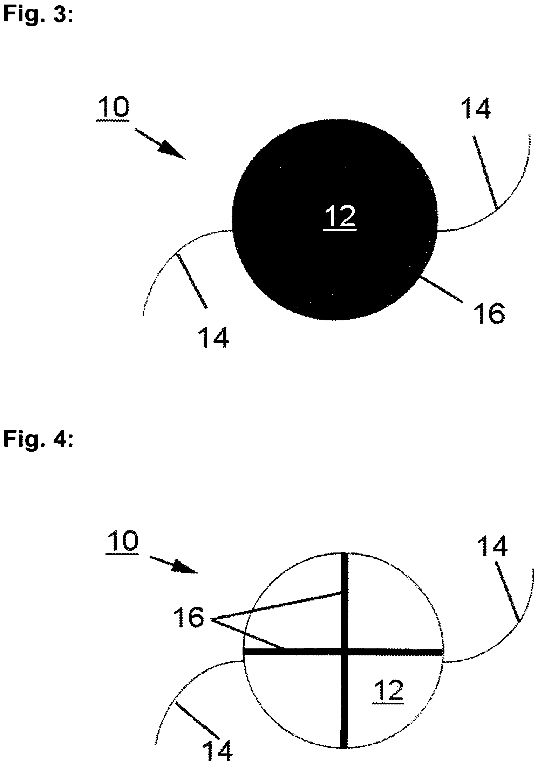

Fig. 3 zeigt eine schematische Darstellung eines ophthalmologischen Implantats 10, nämlich einer Intraokularlinse 10 gemäß einer dritten Ausführungsform. In diesem Ausführungsbeispiel ist der Linsenkörper 12 vollflächig mit einer Markierung 16 versehen. Es ist aber auch möglich, den Linsenkörper 12 nur teilflächig mit einer Markierung 16 (nicht dargestellt) auszubilden. Eine teilflächige Ausgestaltung stellt eine weitere Positions- und Lageangabe zur Verfügung.

Fig. 4 zeigt eine schematische Darstellung eines ophthalmologischen Implantats 10, nämlich einer Intraokularlinse 10 gemäß einer vierten Ausführungsform. Man erkennt, dass der Linsenkörper 12 eine kreuzförmige Markierung 16 aufweist. Eine derartig ausgebildete Markierung 16 ist besonders vorteilhaft, wenn mittels entsprechender Vorrichtungen eine Autofokussierung, eine Bewegungsnachführung oder generell die Darstellung der Orientierung von asymmetrisch ausgebildeten Intraokularlinsen dargestellt und ausgeführt werden soll.

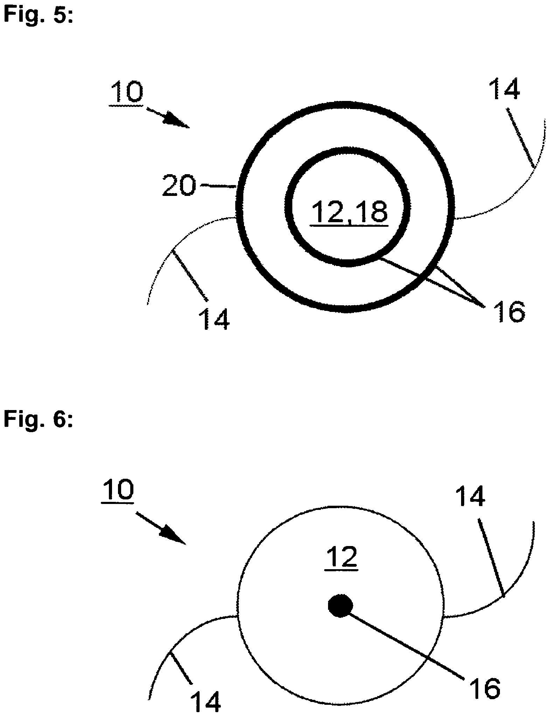

Fig. 5 zeigt eine schematische Darstellung eines ophthalmologischen Implantats 10, nämlich einer Intraokularlinse 10 gemäß einer fünften Ausführungsform. Man erkennt, dass am Linsenkörper 12 zwei konzentrische, ringförmige Markierungen 16 ausgebildet sind. Dabei umgibt eine äußere Markierung 16 den Randbereich 20 des Linsenkörpers 12, die innere Markierung 16 umgibt einen Zentralbereich 18 des Linsenkörpers 12. Auch diese Ausgestaltung der Markierungen 16 eignet sich sehr gut für Autofokussysteme oder Nachführsysteme, die eine mögliche Bewegung oder auch die Position und Lage der Intraokularlinse 10 exakt bestimmen lassen. Am Linsenkörper 12 sind wiederum zwei Befestigungselemente 14 angeordnet.

Fig. 6 zeigt eine schematische Darstellung eines ophthalmologischen Implantats 10, nämlich eine Intraokularlinse 10 gemäß einer sechsten Ausführungsform. Man erkennt, dass im Zentrum des Linsenkörpers 12 eine punktförmige Markierung 16 ausgebildet ist. Des Weiteren sind an dem Linsenkörper 12 wiederum zwei Befestigungselemente 14 ausgebildet.

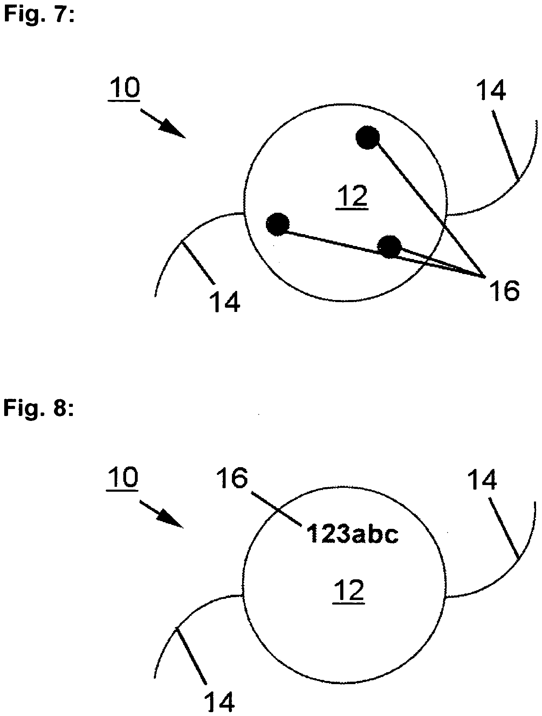

Fig. 7 zeigt eine schematische Darstellung eines ophthalmologischen Implantats 10, nämlich eine Intraokularlinse 10 gemäß einer siebten Ausführungsform. Man erkennt, dass die Intraokularlinse 10 mehrere, nämlich drei Markierungen 16 aufweist, wobei die Markierungen 16 zu einem geometrischen Muster angeordnet sind. An dem Linsenkörper 12 sind wiederum zwei Befestigungselemente 14 ausgebildet. Auch die geometrische Anordnung mehrerer Markierungen 16 eignet sich sehr gut für die Verwendung von Autofokussystemen oder Nachführsystemen.

Fig. 8 zeigt eine schematische Darstellung eines ophthalmologischen Implantats 10, nämlich eine Intraokularlinse 10 gemäß einer achten Ausführungsform. Man erkennt, dass die Markierung 16 aus einer alphanumerischen Reihenfolge bestehend aus Buchstaben und Ziffern besteht. Derartige Markierungen 16 dienen zur Identifikation der Intraokularlinse 10, wobei insbesondere Informationen bezüglich der optischen Eigenschaften der Interokularlinse 10, deren Herstellungsdatum oder deren Materialzusammensetzungen angegeben werden können. Des Weiteren können Informationsdaten bezüglich des Implantats wie zum Beispiel dessen korrekter Positionierung innerhalb des Auges dargestellt werden. Dabei werden insbesondere die Informationsdaten objektgebunden dem realen Bild überlagert und zwar unabhängig von eventuellen Bewegungen des Objekts bzw. des Implantats 10 ("Augmented Reality"). Man erkennt, dass wiederum an dem Linsenkörper 12 zwei Befestigungselemente 14 ausgebildet sind.

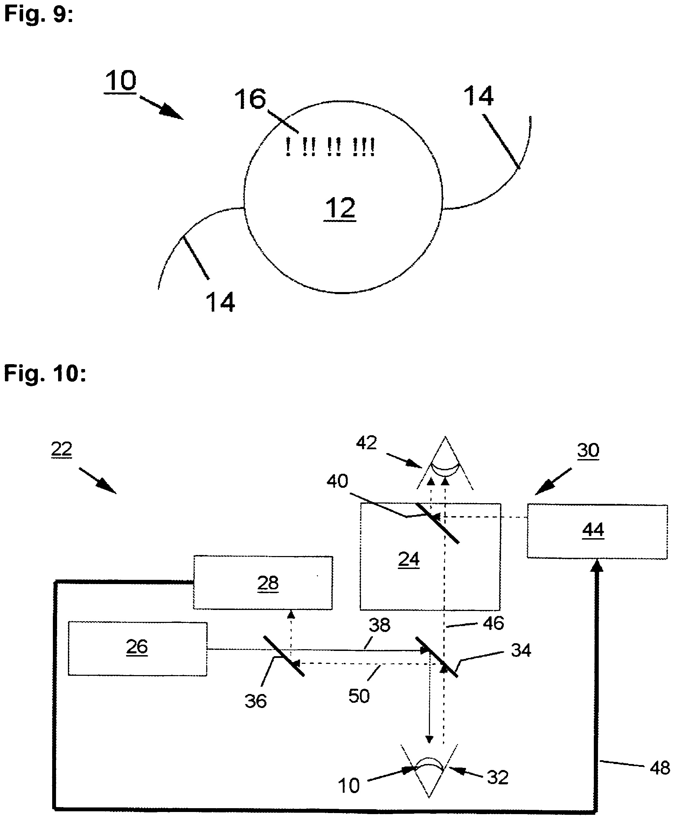

Fig. 9 zeigt eine schematische Darstellung eines ophthalmologischen Implantats 10, nämlich eine Intraokularlinse 10 mit einer Markierung 16, wobei die Markierung 16 aus einem so genannten Barcode besteht der wiederum Informationen bezüglich der Intraokularlinse 10 enthalten kann. Der Barcode wird mittels geeigneter Scanvorrichtungen ausgelesen, so dass die in der Markierung 16 beschriebene Information zur Verfügung gestellt werden kann. Dabei kann die Information zum Beispiel an eine Anzeigevorrichtung oder einen Drucker weitergegeben und dort ausgegeben werden.

Neben den in den Fig. 1 bis 9 beschriebenen Ausführungsbeispielen ist eine Vielzahl weiterer Ausführungsformen des ophthalmologischen Implantats 10 bzw. der Intraokularlinse 10 denkbar. Insbesondere betrifft dies die Ausgestaltung der Markierungen 16 die jede geometrische Form annehmen können. Des Weiteren ist es möglich, dass das ophthalmologische Implantat 10 mindestens zwei mittels unterschiedlicher Farbstoffe hergestellte Markierungen 16 aufweist. Des Weiteren soll der für die Herstellung der Markierung 16 verwendete Farbstoff bioverträglich, insbesondere nicht toxisch sein. Schließlich kann bei allen dargestellten wie auch weiteren Ausführungsformen des ophthalmologischen Implantats 10 die Markierung 16 während der Herstellung und/oder nach der Fertigstellung des ophthalmologischen Implantats 10 ausgebildet werden. Dabei kann zum Beispiel der die Markierung 16 ausbildende Farbstoff vor und/oder nach einer Polymerisation oder Vulkanisation des Linsenmaterials des Implantats 10 in dieses eingebracht werden. Es ist aber auch möglich, dass erst nach der Polymerisation oder Vulkanisation des Linsenmaterials der Farbstoff auf eine Oberfläche des ophthalmologischen Implantats 10 aufgebracht oder in dieses eingebracht wird. Hierfür stehen unterschiedlichste Verfahren zur Verfügung. Beispielhaft sind bei Implantaten 10 aus Kunststoff Mehrfarben-/Mehrkomponenten-Spritzguss-Verfahren, Hinterspritz-/Zwischenschichtspritz-Verfahren, so genannte In-Mould-Dekorierverfahren bzw. Labeling-Verfahren, eine Co- oder Mehrschichten-Extrusion, Laminieren, Bedrucken, Prägen und Plasmaverfahren wie zum Beispiel Chemical Vapor Deposition (CVD) zu nennen. Es ist aber auch möglich, dass die Markierung 16 auf einer Folie aufgebracht ist und die Folie in das ophthalmologische Implantat 10 eingebracht oder auf eine Oberfläche des ophthalmologischen Implantats 10 aufgebracht wird. Des Weiteren ist es möglich, dass Markierungen 16, die an der Oberfläche des Implantats 10 angeordnet sind mit einer Schutzfolie oder einer schützenden Beschichtung überzogen sind. Die Schutzfolie und die Beschichtung dienen insbesondere zur Verhinderung einer Ausspülung durch zum Beispiel das Kammerwasser des Auges.

Fig. 10 zeigt eine schematische Darstellung eines Mikroskopiesystems 22 zur Detektion der Lage und Position und/oder zur Identifikation eines ophthalmologischen Implantats 10, insbesondere einer Intraokularlinse 10 wie sie in den Ausführungsbeispielen gemäß den Fig. 1 bis 9 beschrieben worden ist. Das Mikroskopiesystem 22 umfasst dabei ein Mikroskop 24 mit einer Mikroskopoptik, ein Beleuchtungssystem 26 zur Beleuchtung der aus einem oder mehreren Farbstoffen bestehenden Markierung 16 des in einem Untersuchungsobjekt, insbesondere einem Auge 32 angeordneten Implantats 10. Des Weiteren weist das Mikroskopiesystem 22 eine Kamera 28 zur Aufnahme der außerhalb des für den Menschen sichtbaren Lichtspektrums emittierenden fluoreszierenden oder absorbierenden Markierungen 16 und mindestens eine Auswerteeinheit 44 für mindestens ein von der Kamera 28 aufgenommenes Bild der Markierung bzw. der Markierungen 16 auf. In dem dargestellten Ausführungsbeispiel bestehen die Markierungen 16 wiederum aus einem im nahen Infrarotbereich fluoreszierenden Farbstoff, nämlich dem Farbstoff Indocyaningrün. Aus Fig. 10 wird deutlich, dass das Beleuchtungssystem 26 Licht einer bestimmten Wellenlänge und auch weißes Licht über einen Strahlteiler 34 an das Auge 32 mit der Intraokularlinse 10 abstrahlt. Die von der Intraokularlinse 10 bzw. den Markierungen 16 emittierte Strahlung wird wiederum über den Strahlteiler 34 und einen zweiten Strahlteiler 36 über einen Teilstrahlengang 50 an die Kamera 28 gesandt. Über einen weiteren Teilstrahlengang 46 wird das von dem Auge 32 bzw. der Intraokularlinse 10 aufgenommene Bild dem Auge 42 eines Betrachters über das Mikroskop 24 mit der Mikroskopoptik zur Verfügung gestellt. Hierzu ist der Strahlteiler 34 als teildurchlässiger Spiegel ausgebildet. Die von der Kamera 28 aufgenommenen Bilder werden als Bilddaten über eine Datenleitung 48 an die Auswerteeinheit 44 übermittelt. Die Auswerteeinheit 44 weist ein Koppelungssystem 30 zum Einkoppeln der aufgenommenen Bilder der Markierung 16 in die Mikroskopoptik des Mikroskops 24 auf. Das Koppelungssystem 30 umfasst dabei einen Strahlteiler bzw. Umlenkspiegel 40 der als teildurchlässiger Spiegel ausgebildet ist und das von der Kamera 28 aufgenommene und durch die Auswerteeinheit 44 ausgewertete Bild an das Auge 42 des Betrachters sendet. Dadurch ist es möglich, ein Überlagerungsbild bestehend aus dem von der Kamera 28 aufgenommenen Bild der Markierung 16 und der Abbildung des Objektbereichs, nämlich des beobachteten Implantats 10 und dessen Umgebung für einen Betrachter bereitzustellen. Des Weiteren kann die Auswerteeinheit 44 ein automatisches Nachführsystem zur Erkennung von Bewegungen und/oder der Lage- und Positionsänderungen der Markierung 16 und des Implantats 10 aufweisen. Des Weiteren ist es möglich, dass die Auswerteeinheit ein Autofokussystem umfasst, wobei die Fokussierung der Mikroskopoptik auf das Implantat 10 mittels der von der Kamera 28 aufgenommenen Bilder der Markierung 16 und deren Auswertung erfolgt.

Das Beleuchtungssystem 26 umfasst eine Lichtquelle, wobei die Lichtquelle weißes Licht oder ein Anregungslicht für den Farbstoff der Markierung 16 in einem vorbestimmten Wellenlängenbereich abstrahlt. Als Lichtquellen können zum Beispiel Halogenlampen oder Laserlichtquellen verwendet werden. Des Weiteren ist es möglich, dass das Beleuchtungssystem 26 als Spaltbeleuchtungssystem ausgebildet ist (nicht dargestellt).

Das Mikroskopiesystem 22 sowie die in den Ausführungsbeispielen gemäß den Fig. 1 bis 9 beschriebenen Intraokularlinsen 10 können bei chirurgischen Verfahren, die den Ersatz einer natürlichen Linse eines Auges durch ein Implantat, nämlich eine Intraokularlinse 10 zur Aufgabe haben, verwendet werden. Insbesondere kann bereits während des Einsetzens der Intraokularlinse 10 in den entsprechenden Bereich des Auges, insbesondere die hintere oder vordere Augenkammer, eine Lage- und Positionskontrolle der Intraokularlinse 10 erfolgen. Dadurch ist eine sichere und exakte Positionierung der Intraokularlinse in dem Auge gewährleistet.

## Patentansprüche

1. Ophthalmologisches Implantat, insbesondere Intraokularlinse, mit mindestens einer Markierung (16) hergestellt mit mindestens einem Farbstoff, **dadurch gekennzeichnet, dass**
- der Farbstoff ein fluoreszierender Farbstoff mit einem Emissionsmaximum außerhalb des für den Menschen sichtbaren Lichtspektrums ist und
- der fluoreszierende Farbstoff aus einer der Strukturklassen die Cyanine, Mercocyanine, Phtalocyanine, Rhodamine, Porphyrine, Chlorophylle, Triarylmethane, Xanthene, Oxazine, Thiazine, Safranine, heterozyklische Pyrilium- und Thiapyriliumsalze, Perrylene, Terrylene, Quaterrylene, Quaternele, Azofarbstoffe, Metallkomplexe oder Chinone umfasst, ausgewählt ist,
- oder ein absorbierender Farbstoff mit einem Absorptionsmaximum außerhalb des für den Menschen sichtbaren Lichtspektrums ist, wobei
- der Licht absorbierende Farbstoff ein UV-Absorber aus einer der Strukturklassen die Benzophenone, Benzotriazole, Salicylsäureester, Resorcinmonobenzonate, Oxalsäureanilide, Zimtsäureesterderivate, p-Hydroxybenzoesäureester umfasst und/oder ein IR-Absorber aus einer dieser Strukturklassen, ist,
- jeweils ohne die Lichttransmission des Implantats (10) im visuellen Spektralbereich wesentlich zu beeinflussen.

2. Implantat nach Anspruch 1, **dadurch gekennzeichnet, dass** die mindestens eine Markierung (16) an mindestens einem Befestigungselement (14) und/oder einem Linsenkörper (12) und/oder einem Rand (20) des Linsenkörpers (12) des ophthalmologischen Implantats (10) ausgebildet ist.

3. Implantat nach Anspruch 1 oder 2, **dadurch gekennzeichnet, dass** die Markierung (16) aus Buchstaben und/oder Ziffern und/oder einem Barcode besteht.

4. Implantat nach einem der Ansprüche 1 bis 3, **dadurch gekennzeichnet, dass** die mindestens eine Markierung mindestens ein Befestigungselement (14) und/oder einen Linsenkörper (12) des ophthalmologischen Implantats (10) voll- oder teilflächig markiert.

5. Implantat nach einem der vorhergehenden Ansprüche, **dadurch gekennzeichnet, dass** das ophthalmologische Implantat (10) mindestens zwei mittels unterschiedlicher Farbstoffe hergestellte Markierungen (16) aufweist.

6. Implantat nach einem der vorhergehenden Ansprüche, **dadurch gekennzeichnet, dass** das ophthalmologische Implantat (10) mehrere Markierungen (16) aufweist, wobei die Markierungen (16) in einem oder mehreren geometrischen Mustern angeordnet sind.

7. Implantat nach einem der vorhergehenden Ansprüche, **dadurch gekennzeichnet, dass** die Markierung (16) während der Herstellung und/oder nach der Fertigstellung das ophthalmologischen Implantats (10) ausgebildet ist, und der die Markierung (16) ausbildende Farbstoff vor und/oder nach einer Polymerisation oder Vulkanisation eines Linsenmaterials des ophthalmologischen Implantats (10) in dieses eingebracht ist und/oder nach der Polymerisation oder Vulkanisation des Linsenmaterials auf eine Oberfläche des ophthalmologischen Implantats (10) aufgebracht ist.

8. Implantat nach einem der Ansprüche 1 bis 6, **dadurch gekennzeichnet, dass** die Markierung (16) während der Herstellung und/oder nach der Fertigstellung das ophthalmologischen Implantats (10) ausgebildet ist und die Markierung (16) auf einer Folie aufgebracht ist und die Folie in das ophthalmologische Implantat (10) eingebracht oder auf eine Oberfläche das ophthalmologischen Implantats (10) aufgebracht ist.

9. Implantat nach Anspruch 7 oder 8, **dadurch gekennzeichnet, dass** zumindest der Bereich der Oberfläche des Implantats (10) an dem die mindestens eine Markierung (16) angeordnet ist mit einer Schutzfolie oder einer schützenden Beschichtung überzogen ist.

10. Implantat nach einem der Ansprüche 7 bis 9, **dadurch gekennzeichnet, dass** die Markierung (16) durch ein Präge- oder Druckverfahren auf dem Implantat (10) ausgebildet ist.

## Claims

1. Ophthalmologic implant, in particular intraocular lens, including at least one mark (16) produced with at least one dye, **characterized in that**
- the dye is a fluorescent dye with an emission maximum outside of the light spectrum visible to the human, and
- the fluorescent dye is selected from one of the structural classes including cyanines, merocyanines, phthalocyanines, rhodamines, porphyrines, chlorophylls, triarylmethanes, xanthenes, oxazines, thiazines, safranines, heterocyclic pyrylium and thiapyrylium salts, perrylenes, terrylenes, quaterrylenes, quaterneles, azo dyes, metal complexes or chinones,
- or an absorbing dye with an absorption maximum outside of the light spectrum visible to the human, wherein
- the light absorbing dye is an UV absorber of one of the structural classes including benzophenones, benzotriazoles, salicylic acid esters, resorcinmonobenzoates, oxalic acid anilides, cinnamic acid ester derivatives, p-hydroxy benzoic acid esters, and/or an IR absorber of one of these structural classes,
- each without substantially influencing the light transmission of the implant (10) in the visual spectral range.

2. Implant according to claim 1, **characterized in that** the at least one mark (16) is formed on at least one attachment element (14) and/or a lens body (12) and/or an edge (20) of the lens body (12) of the ophthalmologic implant (10).

3. Implant according to claim 1 or 2, **characterized in that** the mark (16) is composed of letters and/or digits and/or a bar code.

4. Implant according to any one of claims 1 to 3, **characterized in that** the at least one mark marks a full or partial area of at least one attachment element (14) and/or a lens body (12) of the ophthalmologic implant (10).

5. Implant according to anyone of the preceding claims, **characterized in that** the ophthalmologic implant (10) has at least two marks (16) produced by means of different dyes.

6. Implant according to anyone of the preceding claims, **characterized in that** the ophthalmologic implant (10) has several marks (16), wherein the marks (16) are disposed in one or more geometric patterns.

7. Implant according to anyone of the preceding claims, **characterized in that** the mark (16) is formed during the production and/or after completion of the ophthalmologic implant (10), and the dye forming the mark (16) is introduced into a lens material of the ophthalmologic implant (10) before and/or after polymerization or vulcanization of it, and/or is applied to a surface of the ophthalmologic implant (10) after the polymerization or vulcanization of the lens material.

8. Implant according to any one of claims 1 to 6, **characterized in that** the mark (16) is formed during the production and/or after completion of the ophthalmologic implant (10), and the mark (16) is applied to a foil, and the foil is introduced into the ophthalmologic implant (10) or applied to a surface of the ophthalmologic implant (10).

9. Implant according to claim 7 or 8, **characterized in that** at least the region of the surface of the implant (10), on which the at least one mark (16) is disposed, is covered with a protective foil or a protective coating.

10. Implant according to any one of claims 7 to 9, **characterized in that** the mark (16) is formed on the implant (10) by an embossing or printing procedure.

## Revendications

1. Implant ophtalmologique, en particulier lentille intraoculaire, avec au moins un marqueur (16), fabriqué avec au moins un colorant, **caractérisé en ce que**
- le colorant est un colorant fluorescent avec un maximum d'émission en dehors du spectre lumineux, visible pour l'Homme et
- le colorant fluorescent est sélectionné parmi l'une des classes de structures qui comprend la cyanine, la mercocyanine, la phtalocyanine, la rhodamine, la porphyrine, la chlorophylle, le triarylméthane, le xanthène, l'oxazine, la thiazine, la safranine, les sels hétérocycliques de pyrilium et de thiapyrilium, le pérylène, le térylène, le quaterylène, le quaternèle, les colorants azoïques, les complexes métalliques ou la quinone
- ou est un colorant absorbant avec un maximum d'absorption en dehors du spectre lumineux, visible pour l'Homme, moyennant quoi
- le colorant, qui absorbe la lumière, est un absorbeur d'UV de l'une des classes de structures, qui comprend les benzophénones, lesbenzotriazoles, les esters d'acide salicylique, les résorcinol-monobenzoates, les anilides d'acide oxalique, les dérivés de l'ester d'acide cinnamique, des esters d'acide p-hydroxybenzoïque et / ou un absorbeur d'IR de l'une de ces classes de structures,
- sans influer sensiblement, respectivement, sur la transmission de la lumière de l'implant (10) dans le domaine spectral visuel.

2. Implant suivant la revendication 1, **caractérisé en ce que** le au moins un marqueur (16) est formé sur au moins un élément de fixation (14) et / ou un corps de lentille (12) et / ou un bord (20) du corps de lentille (12) de l'implant ophtalmologique (10).

3. Implant suivant la revendication 1 ou 2, **caractérisé en ce que** le marqueur (16) se compose de lettres et / ou de chiffres et / ou d'un code barre.

4. Implant suivant l'une des revendications 1 à 3, **caractérisé en ce que** le au moins un marqueur marque, sur toute la surface ou sur une partie de la surface, au moins un élément de fixation (14) et / ou un corps de lentille (12) de l'implant ophtalmologique (10).

5. Implant suivant l'une des revendications précédentes, **caractérisé en ce que** l'implant ophtalmologique (10) présente au moins deux marqueurs (16), fabriqués au moyen de colorants différents.

6. Implant suivant l'une des revendications précédentes, **caractérisé en ce que** l'implant ophtalmologique (10) présente plusieurs marqueurs (16), moyennant quoi les marqueurs (16) sont agencés en un ou plusieurs modèles géométriques.

7. Implant suivant l'une des revendications précédentes, **caractérisé en ce que** le marqueur (16) est formé pendant la fabrication et / ou après l'achèvement de l'implant ophtalmologique (10) et que le colorant, qui forme le marqueur (16), est introduit dans l'implant ophtalmologique (10) avant et / ou après une polymérisation ou une vulcanisation d'un matériau lenticulaire de l'implant ophtalmologique (10) et / ou est appliqué, après la polymérisation ou la vulcanisation du matériau lenticulaire, sur une surface de l'implant ophtalmologique (10).

8. Implant suivant l'une des revendications 1 à 6, **caractérisé en ce que** le marqueur (16) est formé pendant la fabrication et / ou après l'achèvement de l'implant ophtalmologique (10) et que le marqueur (16) est appliqué sur une feuille et que la feuille est introduite dans l'implant ophtalmologique (10) ou qu'elle est appliquée sur une surface de l'implant ophtalmologique (10).

9. Implant suivant la revendication 7 ou 8, **caractérisé en ce qu'**au moins le secteur de la surface de l'implant (10), sur lequel est agencé le au moins un marqueur (16), est recouvert d'une feuille de protection ou d'une couche protectrice.

10. Implant suivant l'une des revendications 7 à 9, **caractérisé en ce que** le marqueur (16) est formé par le biais d'un procédé d'estampage ou par pression sur l'implant (10).
